# EUROPÄISCHE PATENTSCHRIFT

(11) **EP 3 333 290 B1**
(45) Veröffentlichungstag und Bekanntmachung des Hinweises auf die Patenterteilung: **07.08.2019**
(21) Anmeldenummer: 16203206.4
(22) Anmeldetag: 09.12.2016
(51) Int. Cl.: D04B 1/26, D06P 3/82, D04B 1/12

(54) **KOMPRESSIONSGESTRICKTEIL, KOMPRESSIONSKLEIDUNGSSTÜCK, VERFAHREN ZUM FÄRBEN EINES KOMPRESSIONSGESTRICKTEILS UND VERFAHREN ZUR HERSTELLUNG EINES GEFÄRBTEN KOMPRESSIONSKLEIDUNGSSTÜCKS**
KNIT COMPRESSION FABRIC, COMPRESSION GARMENT, PROCESS FOR DYEING A KNIT COMPRESSION FABRIC AND A METHOD FOR MANUFACTURING A COLOURED KNIT COMPRESSION FABRIC
ÉLÉMENT TRICOTÉ DE COMPRESSION, VÊTEMENT DE COMPRESSION TRICOTÉ, PROCÉDÉ DE TEINTURE D'UN ÉLÉMENT DE COMPRESSION TRICOTÉ ET PROCÉDÉ DE FABRICATION D'UN VÊTEMENT DE COMPRESSION TRICOTÉ ET TEINTÉ

(43) Veröffentlichungstag der Anmeldung: 13.06.2018
(73) Patentinhaber: medi GmbH & Co. KG, 95448 Bayreuth (DE)
(72) Erfinder: Hoffeins, Peter, 95448 Bayreuth (DE); Bauer, Jochen, 95482 Gefrees (DE)
(74) Vertreter: Lindner Blaumeier Patent- und Rechtsanwälte

(56) Entgegenhaltungen:
- FR-A- 1 427 172
- FR-A1- 2 588 890
- US-B1- 7 537 621

## Beschreibung

Die Erfindung betrifft ein Kompressionsgestrickteil aus einem Kompressionsgestrick, ein Kompressionskleidungsstück mit einem solchen Kompressionsgestrickteil, ein Verfahren zum Färben eines solchen Kompressionsgestrickteils und ein Verfahren zur Herstellung eines solchen Kompressionsgestrickteils.

Kompressionsgestrickteile, insbesondere als Kompressionskleidungsstücke und/oder Zusätze zu/Teile von solchen, sind im Stand der Technik bereits weitgehend bekannt. Sie weisen ein häufig auch als kompressives Gestrick bezeichnetes Kompressionsgestrick auf, dessen Aufgabe es ist, gezielt Druck auf eine bestimmte Körperregion, beispielsweise Teile der Muskulatur, auszuüben. Ein Kompressionsgestrick ist im Sinne der vorliegenden Erfindung mithin dazu ausgebildet, am Körper unter Spannung getragen einen bestimmten Druck auf eine bestimmte Körperregion auszuüben. Dabei dient diese Druckausübung üblicherweise therapeutischen Zwecken. Ein Kompressionsgestrick weist hierzu üblicherweise einen als Kompressionsfaden ausgebildeten Schussfaden auf. Bekannte Beispiele für Kompressionskleidungsstücke, die wenigstens ein Kompressionsgestrickteil umfassen, sind Kompressionsstrümpfe und Bandagen.

Kompressionsgestricke weisen also spezielle, sie von anderen Gestricken unterscheidende Eigenschaften, insbesondere hinsichtlich ihrer Struktur, auf.

Nichtsdestotrotz werden Kompressionsstrickteile, insbesondere in Form bzw. als Teil von Kompressionskleidungsstücken, in vielen Anwendungsfällen, beispielsweise als Kompressionsstrumpf und/oder äußerlich anzulegende Bandage, sichtbar getragen, so dass bestimmte Anforderungen an deren Außenansicht bestehen, beispielsweise, um modischen Trends folgen zu können und/oder das Kompressionsgestrickteil eigenen Farbvorlieben anpassen zu können. Um gemusterte oder anderweitig farblich gestaltete Kompressionsgestrickteile zu schaffen, sind im Stand der Technik bereits verschiedene grundsätzliche Vorgehensweisen bekannt. So ist es beispielsweise möglich, bereits bei der Herstellung des Kompressionsgestricks wenigstens zwei unterschiedliche Grundfäden zu verwenden, die wenigstens teilweise bereits vorgefärbt sind, um so ein Kompressionsgestrickteil einer bestimmten Farbe bzw. Farbkombination zu erhalten. Ein derartiges gefärbtes Kompressionsgestrick wird in der FR 2 588 890 A1 beschrieben. Bekannt ist es zudem, bei zwei Grundfäden nur einen vorgefärbt zu verwenden, einen anderen aber nachzufärben. Hierfür können unterschiedliche Färbeeigenschaften aufweisende Grundfasern verwendet werden. Diese Vorgehensweise ist letztlich jedoch nachteilhaft, da die Farben für das fertige Produkt (zumindest teilweise) festgelegt sind, und für Bestellungen entsprechend in hinreichendem Maße vorgehalten werden müssen. Verkaufen sich nun manche Farben/Farbkombinationen schlechter als andere Farben/Farbkombinationen, müssen gegebenenfalls fertige Produkte teilweise entsorgt und teilweise nachproduziert werden.

Für Kompressionsgestricke werden häufig Polyamid-Kunstfasern verwendet. Bezüglich solcher Polyamid-Kunstfasern wird in DE 1 619 575 A eine einbadige Mehrfarbenfärbung von Textilien vorgeschlagen. Beispielsweise durch Einkondensieren bestimmter Substanzen in das Polyamid bildende Ausgangsmaterial kann das Farbstoffaufnahmevermögen der Polyamide extrem verändert werden, wobei konkret vorgeschlagen wird, basisch oder sauer modifizierte Polyamide mit einer Farbflotte, die nebeneinander wenigstens einen sauren und basischen Farbstoff enthält, gleichzeitig zwei- oder mehrfarbig zu färben.

Diese Ausgestaltung ist dahingehend nachteilhaft, dass selbst bei Modifikationen der Polyamide es noch zu Restanlagerungen der entsprechenden Farbstoffe an dem jeweils anderen Polyamid kommen kann, so dass Verschmutzungseffekte auftreten können, die den optischen Eindruck des fertigen Kompressionsgestrickteils bzw. Kompressionskleidungsstücks stark negativ beeinflussen.

Die US 7 537 621 B1 hingegen beschreibt ein Verfahren, in welchem zwei unterschiedliche Fasern eines Vlieses aus Nylon und Polyester gemeinsam im selben Färbebad im gleichen Farbton und demzufolge nicht unterschiedlich eingefärbt werden sollen. Die FR 1 427 172 A zielt zwar auf die Bereitstellung eines ungefärbten Strumpfes ab, schlägt aber ein zweibadiges Färbeverfahren zum Einfärben der Polyamid- und Polyesterfäden vor. Der Erfindung liegt daher die Aufgabe zugrunde, eine für Kompressionsgestricke geeignete, demgegenüber verbesserte Möglichkeit zum einbadigen mehrfarbigen Färben bereitzustellen.

Zur Lösung dieser Aufgabe sind erfindungsgemäß ein Kompressionsgestrickteil gemäß Anspruch 1, ein Kompressionskleidungsstück gemäß Anspruch 9, ein Verfahren zum Färben eines Kompressionsgestrickteils nach Anspruch 10 und ein Verfahren zur Herstellung eines gefärbten Kompressionsgestrickteils oder Kompressionskleidungsstücks gemäß Anspruch 12 vorgesehen.

Das erfindungsgemäße Kompressionsgestrickteil zeichnet sich dadurch aus, dass es aus einem Kompressionsgestrick besteht, welches umfasst:
- einen ungefärbten ersten Grundfaden und einen ungefärbten zweiten Grundfaden, wobei der erste Grundfaden zumindest an seiner Oberfläche aus einer Polyamid-Kunstfaser, die einen ersten Farbstoff annimmt, einen zweiten Farbstoff jedoch nicht, und der zweite Grundfaden zumindest an seiner Oberfläche aus einer Polyester-Kunstfaser, die den zweiten Farbstoff annimmt, den ersten Farbstoff jedoch nicht, bestehen, und
- einen als Kompressionsfaden ausgebildeten ungefärbten Schussfaden.

Es wird mithin vorgeschlagen, ein Kompressionsgestrick aus zunächst noch ungefärbten Grundfäden aufzubauen, von denen einer einen Musterfaden bilden kann. Die Grundfäden unterscheiden sich durch ihre bezüglich ihrer Oberfläche gegebene Farbstoffaffinität derart, dass von jeder der Grundfasern ein Farbstoff angenommen wird, der von der anderen Grundfaser nicht angenommen wird. Dies wird dadurch erreicht, dass zum einen Polyamid-Kunstfasern (PA), zum anderen aber auch Polyester-Kunstfasern (PES) verwendet werden, wobei es bevorzugt wird, kationisch färbbares Polyester (oft auch "kationisches Polyester") zu verwenden. Durch die Verwendung dieser beiden unterschiedlichen Kunstfasern, die die Oberfläche der Grundfäden zumindest im Wesentlichen bilden, liegen derart unterschiedliche Färbeeigenschaften vor, dass keine Verschmutzungseffekte mehr auftreten, das bedeutet, eine Anlagerung des ersten Farbstoffs an die Polyester-Kunstfaser bzw. des zweiten Farbstoffs an die Polyamid-Kunstfaser ist ausgeschlossen oder zumindest so gering, dass das keine optischen Beeinträchtigungen auftreten.

Das noch ungefärbt vorliegende Kompressionsgestrickteil kann nun im später noch näher zu erläuternden erfindungsgemäßen Färbeverfahren einbadig in einer die beiden Farbstoffe enthaltenden Flotte je nach Bedarf auf einfache Art und Weise gefärbt werden. Mit anderen Worten ist es ausreichend, ungefärbte Kompressionsgestrickteile zu bevorraten bzw. einzulagern, nachdem diese mit wenig Zeitaufwand und geringem Wasserverbrauch, insbesondere also bei geringen Kosten, nach Bedarf dann auch mehrfarbig gefärbt werden können. Das bedeutet, es sind nicht lediglich Ton-in-Ton-Effekte möglich. Es ist weder eine Bevorratung/Lagerhaltung von Farbgarnen noch von bereits gefärbten Kompressionsgestrickteilen bzw. Kompressionskleidungsstücken notwendig. Produktionsmaschinen der Strickerei bzw. Konfektion müssen bei Muster- und Farbwechsel nicht gerüstet werden. Die mit der vorliegenden Erfindung mögliche Farbgestaltung geht über einen Ton-in-Ton-Effekt (hell/dunkel) weit hinaus. Nachdem Polyester, insbesondere kationisch färbbares Polyester, verwendet wird, können die Färbetemperaturen niedrig gehalten werden, insbesondere unter 97 °C, wobei das Färbeverfahren aufgrund der Möglichkeit zur einbadigen Färbung ebenso kosteneffektiv gehalten werden kann.

Dabei muss das Kompressionsgestrickteil nicht zwangsläufig ein Kompressionskleidungsstück bilden bzw. Teil eines Kompressionskleidungsstücks sein, sondern es ist durchaus auch denkbar, dass das Kompressionsgestrickteil ein Zubehör bzw. Accessoire zu einem Kompressionskleidungsstück bildet bzw. diesem zugehörig ist, dass auch Zubehör und Accessoires farblich kompatibel angepasst werden können. Das mögliche einbadige Färben reduziert zudem die notwendigen Ressourcen (Wasser/Energie/Arbeitszeit), so dass die geringen Färbekosten bei hohen Farbechtheiten gegeben sind.

Dabei sei an dieser Stelle nochmals angemerkt, dass unter einem Kompressionsgestrick bzw. kompressiven Gestrick im Rahmen der vorliegenden Erfindung ein solches verstanden werden soll, welches bei Applizierung auf eine Körperregion auf diese gezielt einen bestimmten, insbesondere therapeutisch wirkenden Druck ausübt. Beispielsweise im Fall von Kompressionsstrümpfen als Kompressionskleidungsstücken bzw. das Kompressionskleidungsstück bildenden Kompressionsgestrickteilen sind beispielsweise bestimmte Kompressionsklassen normiert, die die Kompressionswirkung auf Körperregionen entsprechend beschreiben. Bildet das Kompressionsgestrickteil mithin beispielsweise als Kompressionskleidungsstück einen Kompressionsstrumpf, kann dieser einer bestimmten Kompressionsklasse (CCL) zugeordnet sein, die üblicherweise einem Druckwert an der Fessel entspricht.

Eine besonders bevorzugte Weiterbildung der vorliegenden Erfindung sieht vor, dass der Kompressionsfaden einen mit derselben Kunstfaser wie einer der Grundfäden, insbesondere der Polyamid-Kunstfaser des ersten Grundfadens, umwundenen Fadenkern, insbesondere aus Elastan, umfasst. In diesem Fall besteht mithin auch die Oberfläche des Kompressionsfadens aus einer der Kunstfasern, so dass der Kompressionsfaden beim Färben entsprechend mitgefärbt wird, bei Verwendung von Polyamid-Kunstfaser zur Umwindung des Kompressionsfadens also mit dem ersten Farbstoff, bei Umwindung mit Polyester-Kunstfaser also mit dem zweiten Farbstoff. Damit kann erreicht werden, dass letztlich im Kompressionsgestrick nur zwei Farben vorkommen.

Eine besonders vorteilhafte Ausgestaltung ergibt sich, wenn der Kompressionsfaden eine unterschiedliche Farbstoffaffinität im Vergleich zu dem dieselbe Kunstfaser aufweisenden Grundfaden bezüglich des entsprechenden Farbstoffs aufweist, so dass bei Färben ein Ton-in-Ton-Effekt hinsichtlich des entsprechenden Farbstoffs entsteht. Die Präsenz des Kompressionsfadens bei Kompressionsgestricken kann mithin ausgenutzt werden, um einen zusätzlichen Designfreiheitsgrad zu erhalten, insbesondere also eine Ton-in-Ton-Variation bezüglich wenigstens einer der Farben zu erhalten. So kann beispielsweise dann, wenn der Kompressionsfaden an seiner Oberfläche Polyamid aufweist, ein Ton-in-Ton-Effekt, mithin unterschiedliche Helligkeiten der Farbe, bezüglich des ersten Farbstoffs erreicht werden, was entsprechend für die Polyester-Kunstfaser und den zweiten Farbstoff gilt, falls der Kompressionsfaden Polyester-Kunstfasern als Umwindung aufweist.

Konkret kann hierbei vorgesehen sein, dass die unterschiedliche Farbstoffaffinität durch eine unterschiedliche Oberflächenstruktur des Kompressionsfadens im Vergleich zu dem dieselbe Kunstfaser aufweisenden Grundfaden realisiert ist, insbesondere durch eine unterschiedliche Zahl von Umwindungen eines jeweiligen Fadenkerns mit der entsprechenden Kunstfaser und/oder durch unterschiedliche Vorspannungen der Fäden. Konkret kann die unterschiedliche Farbstoffaffinität mithin an unterschiedlichen Oberflächenstrukturen des Kompressionsfadens und des dieselben Kunstfaser verwendenden Grundfadens festgemacht werden, welche beispielsweise dadurch erreicht werden kann, dass die Zahl der Umwindungen des Fadenkerns zwischen dem Grundfaden und dem Kompressionsfaden variiert wird. So entsteht durch die unterschiedliche Anhaftung des entsprechenden Farbstoffs insgesamt ein zusätzlicher Designfreiheitsgrad, mit dem weitere optisch anspruchsvolle Mustergebungen und Farbgebungen realisiert werden können.

Dabei sei an dieser Stelle noch darauf hingewiesen, dass unterschiedliche Farbeindrücke auch durch Variationen der Geometrie des mit dem selben Farbstoff wie einer der Grundfäden färbbaren Kompressionsfadens im Vergleich zu dem entsprechenden Grundfaden erreicht werden kann. Beispielsweise kann also vorgesehen sein, dass sich der Kompressionsfaden und ein mit dem gleichen Farbstoff wie der Kompressionsfaden färbbarer Grundfaden in ihrer Geometrie, insbesondere ihrem Querschnitt, unterscheiden, Beispielsweise kann die Veränderung der Dicke des Schussfadens zu einer optisch anderen, gewünschten Farbwirkung führen.

Dabei sei angemerkt, dass es selbstverständlich auch denkbar ist, eine gleiche Farbstoffaffinität des Kompressionsfadens und des entsprechenden Grundfadens bezüglich des entsprechenden Farbstoffs herzustellen, wenn gewünscht ist, dass eine Zweifarbigkeit ohne Ton-in-Ton-Effekte entsteht.

Eine Struktur mit einem umwundenen Kern muss dabei nicht allein für den Kompressionsfaden gegeben sein, sondern kann auch für die Grundfäden realisiert werden. So kann vorgesehen sein, dass wenigstens einer der Grundfäden einen mit der entsprechenden Kunstfaser umwundenen Fadenkern, insbesondere aus Elastan, aufweist.

Einer der Grundfäden kann zweckmäßigerweise ein mustergebender Musterfaden sein, der auf einer Sichtseite des Kompressionsgestricks vor dem anderen Grundfaden liegt. Damit bestimmt der Musterfaden, wie grundsätzlich bekannt, hauptsächlich die Musterung. Beispielsweise kann zur konkreten Umsetzung vorgesehen sein, dass der den Musterfaden bildende Grundfaden in einer Rechts-Links-Grundbindung und der andere Grundfaden in einer Rechts-Rechts-Grundbindung gestrickt sind. Selbstverständlich sind diesbezüglich hinsichtlich des gewünschten Musters verschiedene Möglichkeiten denkbar, um spezielle Designvorgaben umzusetzen, wobei die grundsätzlich aus dem Stand der Technik bekannten Strickvariationen eingesetzt werden können.

Ein erfindungsgemäßes Kompressionskleidungsstück umfasst wenigstens ein Kompressionsgestrickteil der erfindungsgemäßen Art, wobei insbesondere das Kompressionskleidungsstück, bevorzugt ein Kompressionsstrumpf und/oder eine Bandage, auch durch das Kompressionsgestrickteil gebildet sein kann. Die Ausführungen bezüglich des Kompressionsgestrickteils gelten selbstverständlich analog für das Kompressionskleidungsstück fort.

Die Erfindung betrifft ferner ein Verfahren zum Färben eines erfindungsgemäßen Kompressionsgestrickteils in einem einbadigen Färbevorgang, wobei das Verfahren folgende Schritte umfasst:
- Vorbehandlung des Kompressionsgestrickteils,
- Zugabe des ersten Farbstoffs für die Polyamid-Kunstfasern in eine verwendete Flotte und Färben des Kompressionsgestrickteils bei wenigstens einer ersten Temperatur,
- nach Ablauf einer ersten Färbezeitspanne Zugabe des zweiten Farbstoffs einer von der Farbe des ersten Farbstoffs unterschiedlichen Farbe zu der Flotte und Aufheizen der Flotte,
- Färben bei wenigstens einer zweiten Temperatur, die höher ist als die erste Temperatur, aber gleich oder kleiner als 97 °C ist, in einer zweiten Färbezeitspanne, und
- Nachbehandlung des Kompressionsgestrickteils,
wobei zur Trennung des ersten und des zweiten Farbstoffs ein Farbstofftrennmittel verwendet wird.

Im erfindungsgemäßen Verfahren findet also zunächst eine Vorbehandlung des Kompressionsgestrickteils statt, die im Wesentlichen der grundsätzlich bekannten, üblichen Vorbehandlung bei einer Polyamid-Färbung entspricht. Insbesondere wird hier der verwendeten Flotte, in die das zu färbende Kompressionsstrickteil eingetaucht ist, bereits ein Netzmittel zugegeben, welches für eine bestimmte Zeitspanne, beispielsweise 20 bis 30 Minuten, auf das Kompressionsgestrickteil einwirken kann. Auch die Nachbehandlung kann wie bei den üblichen, grundsätzlich bekannten Polyamid-Färbeverfahren vorgenommen werden und umfasst beispielsweise das Spülen und Trocknen des Kompressionsgestrickteils.

Zweckmäßigerweise wird das Farbstofftrennmittel, welches die beiden Farbstoffe getrennt hält, bereits vor Zugabe des ersten Farbstoffs in die Flotte zugegeben, insbesondere gemeinsam mit wie üblich verwendeten Färbehilfsmitteln. Bevor der erste Farbstoff, der auf die Polyamid-Kunstfasern wirkt, zugegeben wird, wird üblicherweise der pH-Wert der Flotte entsprechend angepasst, insbesondere durch Zugabe eines Säurespenders, so dass sich, insbesondere mit Erreichen der Endtemperatur, ein pH-Wert von etwa drei einstellt. Dieser kann über den gesamten Färbevorgang beibehalten werden.
Zunächst wird dann der erste, auf die Polyamid-Kunstfaser wirkende Farbstoff in das Färbeband zugegeben, wobei üblicherweise wenigstens eine erste Temperatur beibehalten bzw. eingestellt wird, wobei insbesondere auch während des Färbevorgangs mit dem ersten Farbstoff, also der ersten Färbezeitspanne, bereits ein Aufheizen hin zur wenigstens einen zweiten Temperatur oder einer Zwischentemperator erfolgen kann. Nach dem Ende der ersten Färbezeitspanne wird der zweite Farbstoff, der auf die Polyester-Kunstfaser wirkt, zugegeben. Nachdem ein mehrfarbiges Färben stattfinden soll, unterscheidet sich die vom ersten Farbstoff erzeugte Farbe von der des zweiten Farbstoffs. In der zweiten Färbezeitspanne wird bei Temperaturen von maximal 97 °C, mithin ohne Druck, gefärbt. Dabei kann die erste Temperatur beispielsweise in einem Intervall von 50 bis 80 °C liegen, die zweite Temperatur beispielsweise in einem Intervall von 80 bis 97 °C.

Wie grundsätzlich bekannt ist es zweckmäßig, wenn während der Färbezeitspannen das Kompressionsgestrickteil in der Flotte bewegt wird, um das Anfärben zu begünstigen.

Insgesamt wird es durch das erfindungsgemäße Färbeverfahren, wie bereits dargelegt wurde, ermöglicht, letztlich auf Anforderung eingelagerte, fertige Kompressionsgestrickteile bzw. Kompressionskleidungsstücke herzunehmen und kosteneffektiv in den gewünschten Farben mehrfarbig einzufärben. Dabei können niedrige Färbetemperaturen gehalten werden und es ist nur ein einziges Färbebad erforderlich, so dass die notwendigen Ressourcen (Wasser/Energie/Arbeitszeit) reduziert sind. Auch ansonsten gelten selbstverständlich die Ausführungen bezüglich des Kompressionsgestrickteils fort.

In konkreter Ausgestaltung des Färbeverfahrens kann vorgesehen sein, dass als erster Farbstoff ein anionischer Farbstoff zur Färbung der Polyamid-Kunstfasern und als zweiter Farbstoff ein kationischer Farbstoff zur Färbung der kationisch färbbar ausgebildeten Polyester-Kunstfasern verwendet wird. Es werden als Direktfarbstoffe mithin Säurefarbstoffe und basische Farbstoffe eingesetzt, wie sie grundsätzlich bekannt und frei erhältlich sind. Aufgrund der gänzlich unterschiedlichen Kunstfaserstruktur werden Verschmutzungseffekte dennoch vermieden.

In zweckmäßiger Ausgestaltung des erfindungsgemäßen Färbeverfahrens ist vorgesehen, dass als Farbstofftrennmittel ein Egalisierungsmittel verwendet wird. Insbesondere kann bei der Verwendung von anionischen und kationischen Farbstoffen (also Säurefarbstoffen und basischen Farbstoffen) als Egalisierungsmittel das unter dem Markennamen "Rucogal CDP" vertriebene Produkt der Firma Rudolf GmbH, Geretsried (Deutschland), verwendet werden. Hierbei handelt es sich um ein Spezialprodukt auf der Grundlage einer Zubereitung aus Fettalkoholethoxylat, Fettsäureester, Alkylarylsulfonat und Alkylaminethoxylat, anionaktiv, das für das einbadige Färben mit basischen Farbstoffen und Säurefarbstoffen die Farbstoffe trennend wirkt und zudem eine sehr gute Egalisierungswirkung für beide Kunstfasern bietet. Bei Versuchen hat sich hier überraschend gezeigt, dass dieses Egalisierungsmittel auch als Farbstofftrennmittel hervorragend wirkt und eine Wechselwirkung des ersten Farbstoffs und des zweiten Farbstoffs im Wesentlichen verhindert.

Die Erfindung betrifft schließlich auch ein Herstellungsverfahren für ein gefärbtes Kompressionsgestrickteil, insbesondere auch eines durch das Kompressionsgestrickteil gebildeten Kompressionskleidungsstücks, welches folgende Schritte umfasst:
- Bereitstellen und Lagern eines ungefärbten Kompressionsgestrickteils der erfindungsgemäßen Art, und
- bei Erhalt einer zwei zu verwendende Farben beschreibenden Farbanforderung Färben des Kompressionsgestrickteils entsprechend der Farbanforderung in einem erfindungsgemäßen Färbeverfahren.

Erfindungsgemäß ist es also nicht notwendig, Kompressionsgestrickteile verschiedener Farben und Farbkombinationen ständig auf Lager vorrätig zu haben, sondern es können die noch ungefärbten Kompressionsgestrickteile eingelagert werden, bis bekannt ist, in welchen Farbkombinationen, beschrieben durch die Farbanforderung, diese benötigt werden. Entsprechend der Farbanforderung ist es dann möglich, das erfindungsgemäße Färbeverfahren einzusetzen und kosteneffektiv und aufwandsarm bei hoher Farbechtheit ein entsprechend gefärbtes Kompressionsgestrickteil herzustellen und beispielsweise einem Kunden bereitzustellen. Insgesamt müssen so deutlich weniger Kompressionsgestrickteile auf Lager gehalten werden und es existiert deutlich weniger überzählige Ware in Farbkombinationen, die, wie sich erst später herausstellt, weniger gewünscht sind.

Weitere Vorteile und Einzelheiten der vorliegenden Erfindung ergeben sich aus den im Folgenden beschriebenen Ausführungsbeispielen sowie anhand der Zeichnungen. Dabei zeigen:
- Fig. 1: eine Prinzipskizze eines erfindungsgemäßen Kompressionsgestrickteils,
- Fig. 2: eine erste Ansicht des Kompressionsgestricks des Kompressionsgestrickteils gemäß Fig. 1,
- Fig. 3: eine zweite Ansicht des Kompressionsgestricks des Kompressionsgestrickteils gemäß Fig. 1,
- Fig. 4: eine Färbekurve eines erfindungsgemäßen Färbeverfahrens, und
- Fig. 5: einen Ablaufplan des erfindungsgemäßen Herstellungsverfahrens.

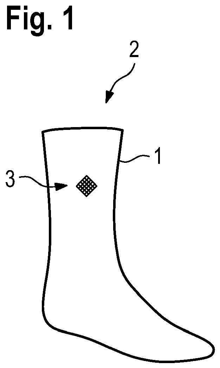

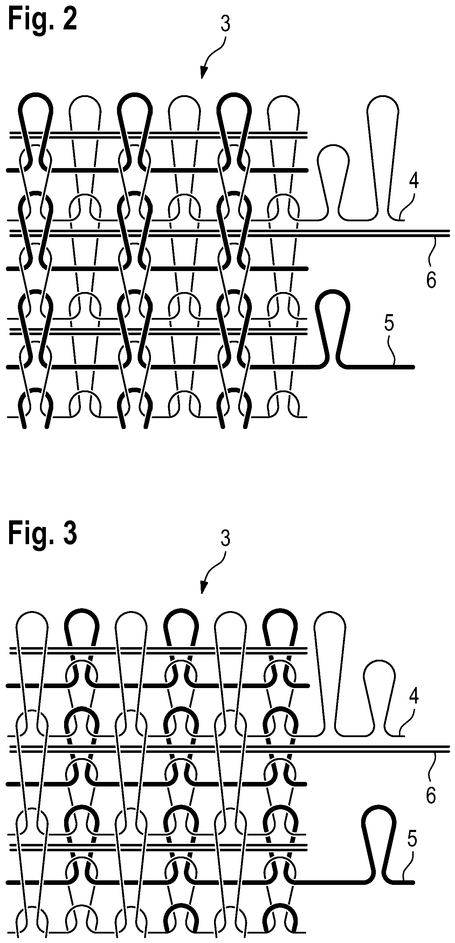

Fig. 1 zeigt eine Prinzipskizze eines erfindungsgemäßen Kompressionsgestrickteils 1, das vorliegend als Kompressionskleidungsstück einen Kompressionsstrumpf bildet. Der Kompressionsstrumpf 2 entfaltet durch das verwendete, in Fig. 1 nur angedeutete und in den Fig. 2 und 3 näher dargestellte Kompressionsgestrick 3, aus dem er besteht, eine bestimmte, gewollte Kompressionswirkung auf die bedeckten Körperregionen, die insbesondere therapeutischen Zwecken dient. Der Kompressionsstrumpf ist insbesondere einer der bekannten, normierten, vier Kompressionsklassen zuzuordnen.

Die Fig. 2 und 3 zeigen das Kompressionsgestrick 3 genauer, wobei vorliegend jeweils die rechte Warenseite gezeigt ist, wobei Fig. 2 eine Ansicht aus Sichtrichtung, also auf die Sichtseite, ist, Fig. 3 eine Ansicht auf die Innenseite des Kompressionsstrumpfes 2 .

Wie Fig. 2 zeigt, umfasst das Kompressionsgestrick 3 drei Arten von Fäden, nämlich einen ersten Grundfaden 4, einen zweiten, einen Musterfaden bildenden Grundfaden 5 und einen Kompressionsfaden 6, die zur Verdeutlichung ihres Verlaufes über den Ausschnitt des Kompressionsgestricks 3 auf der rechten Seite verlängert sind. Ersichtlich liegt der den Musterfaden bildende Grundfaden 5 gemäß Fig. 2 zur Sichtseite hin außen, also insbesondere oberhalb der Kompressionsfäden 6, die als Schussfäden eingebracht sind. Der Grundfaden 4 verläuft hinter dem Kompressionsfaden 6. Entsprechend, vgl. Fig. 3, ist von der Innenseite betrachtet zu erkennen, dass der Grundfaden 5 hinter den Kompressionsfäden 6, also nach außen, liegt.

Der erste Grundfaden 4 und der zweite Grundfaden 5 sind dabei unterschiedlich ausgebildet. Zwar weisen beide als Fadenkern einen Elastankern auf, jedoch ist der Grundfaden 4 von einer Polyamid-Kunstfaser umwunden, der Grundfaden 5 von kationisch färbbarem Polyester. Damit weisen die Grundfäden 4, 5 unterschiedliche Eigenschaften hinsichtlich der Färbbarkeit auf. So ist der Grundfaden 4 aufgrund der Polyamid-Kunstfaser nur durch anionische Farbstoffe (Säurefarbstoffe) als erster Farbstoff färbbar, kationische Farbstoffe als zweiter Farbstoff werden nicht angenommen. Dagegen ist der Grundfaden 5 nur durch kationische Farbstoffe als zweite Farbstoffe färbbar, da seine Oberfläche aus kationisch färbbarer Polyester-Kunstfaser besteht. Diese wiederum nimmt anionische Farbstoffe nicht an.

Vorliegend ist auch der Kompressionsfaden 6 in dieses Färbbarkeitskonzept eingebunden. Nachdem der Kompressionsfaden 6 selbst aus mit Polyamid-Kunstfaser umwundenen Elastan besteht, nimmt er mithin auch anionische Farbstoffe, mithin die ersten Farbstoffe, an. Dabei unterscheiden sich der Kompressionsfaden 6 und der Grundfaden 4 im vorliegenden Ausführungsbeispiel auch hinsichtlich ihrer Oberflächenstruktur, was durch die Anzahl an Umwindungen mit der Polyamid-Kunstfaser und/oder unterschiedliche Vorspannungen erreicht wird. Auf diese Weise entsteht bei der Färbung mit dem ersten Farbstoff ein Ton-in-Ton-Effekt, nachdem der Kompressionsfaden 6 und der Grundfaden 4 den ersten Farbstoff unterschiedlich stark annehmen. Es sind auch Ausführungsbeispiele denkbar, in denen für eine gleiche Oberflächenstruktur bei dem Kompressionsfaden 6 und dem Grundfaden 4 gesorgt ist.

Im Rahmen eines erfindungsgemäßen Färbeverfahrens wird das Kompressionsgestrickteil unter Verwendung eines ersten Farbstoffes und eines zweiten Farbstoffes gefärbt, wobei Farbstoffe unterschiedlicher Farben eingesetzt werden. Dabei findet nach einem grundsätzlich bekannten Vorbereitungsschritt ein einbadiges Färben insgesamt ohne Druck statt, wonach sich der grundsätzlich bekannte Nachbearbeitungsschritt anschließt.

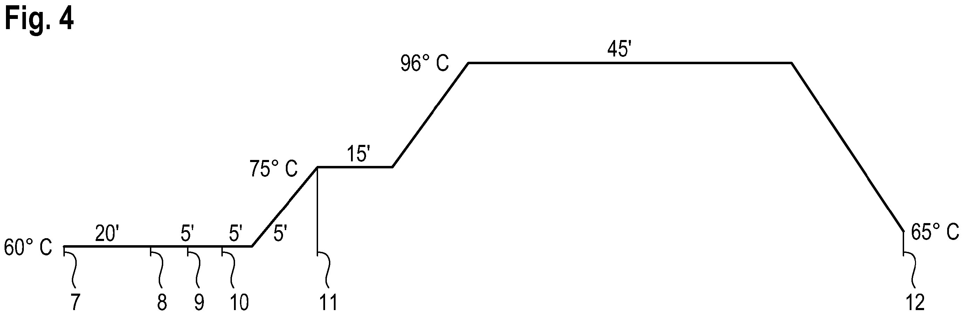

Eine Färbekurve eines Ausführungsbeispiels des erfindungsgemäßen Färbeverfahrens zeigt Fig. 4.

Dort beginnt der eigentliche Färbevorgang unter Nutzung einer entsprechenden Flotte zu einem Zeitpunkt 7, zu dem der Flotte, in der das Kompressionsgestrickteil 1 bereits befindlich ist, ein Netzmittel oder das Kompressionsgestrickteil der vorbereiteten Flotte mit dem Netzmittel zugegeben wird. Die Flotte hat dabei zunächst eine Temperatur von 60 °C. Das Kompressionsgestrickteil wird für 20 Minuten dem Netzmittel ausgesetzt, ehe zu einem Zeitpunkt 8 zum einen grundsätzlich bekannte Färbehilfsmittel zugegeben werden, zusätzlich aber auch ein Farbstofftrennungsmittel. Als Farbstofftrennungsmittel wird hier ein spezielles Egalisierungsmittel verwendet, insbesondere das bereits erwähnte Rucogal CDP. Zu einem Zeitpunkt 9 erfolgt dann die Zugabe von Säurespendern, die den pH-Wert der Flotte einstellen sollen. Dabei wirkt der Säurespender derart, dass im Laufe der Zeit mit steigender Temperatur Säure freigesetzt wird, so dass mit Erreichen der Endtemperatur von hier 96 °C ein pH-Wert von drei erreicht wird.

Zu einem Zeitpunkt 10 wird dann schließlich der erste Farbstoff für die Polyamid-Kunstfaser zugegeben. Die Temperatur von 60 °C wird noch für fünf Minuten gehalten, ehe ein Aufheizen auf eine Temperatur von 75 °C über weitere fünf Minuten stattfindet. Während der gesamten Zeit wird das Kompressionsgestrickteil 1 in der Flotte bewegt. Zwischen den Zeitpunkten 10 und 11 liegt eine erste Färbezeitspanne. Bereits bei schwach saurem pH-Wert und 60 °C zieht der als anionischer Farbstoff (Säurefarbstoff) ausgebildete erste Farbstoff auf die Polyamid-Kunstfaser auf.

Zum Zeitpunkt 11 wird dann der zweite Farbstoff für die kationische Polyester-Kunstfaser hinzugefügt, so dass dann der anionische erste Farbstoff und der kationische zweite Farbstoff beide in der Flotte vorliegen, es jedoch nicht zu relevanten Wechselwirkungen kommt, da dies durch das FarbstoffTrennungsmittel verhindert wird. In der nun beginnenden zweiten Färbezeitspanne wird zunächst für 15 Minuten die Temperatur von 75° gehalten, ehe die Temperatur auf 96 °C erhöht wird, wonach die zweite Färbezeitspanne für 45 Minuten fortgesetzt wird, in denen das Kompressionsgestrickteil weiterhin in der Flotte bewegt wird. Nachdem die zweite Temperatur während der zweiten Färbezeitspanne immer unterhalb von 97 °C bleibt, wird mithin ohne Druck gefärbt. Der kationische zweite Farbstoff benötigt einen tieferen pH-Wert, der durch Freigabe von Säure durch den Säurespender bereits bei 75 °C auftritt, so dass das Aufziehen des zweiten Farbstoffs auf die Polyester-Kunstfaser beginnt. Im Laufe der Zeit wird mit der Temperaturerhöhung durch den Säurespender mehr Säure freigegeben, was langsam das Aufziehvermögen der Farbstoffe erhöht, bis der End-pH-Wert von drei erreicht ist.

Nach einem Abkühlvorgang markiert der Zeitpunkt 12 den Beginn der erwähnten Nachbehandlung des Kompressionsgestrickteils 1.

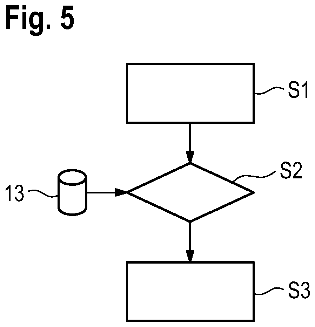

Fig. 5 zeigt einen Ablaufplan eines Ausführungsbeispiels für ein Herstellungsverfahren eines gefärbten Kompressionsgestrickteils 1. In einem Schritt S1 wird das noch ungefärbte Kompressionsgestrickteil 1 bereitgestellt, und eingelagert. In einem Schritt S2 wird überprüft, ob bereits eine Farbanforderung 13 für das Kompressionsgestrickteil vorliegt, die beim Färben zu verwendende Farben beschreibt. Liegt diese vor, wird das Kompressionsgestrickteil 1 wie bezüglich des Färbeverfahrens beschrieben, im Schritt S3 entsprechend der Farbanforderung 13 gefärbt.

## Patentansprüche

1. Kompressionsgestrickteil (1) aus einem Kompressionsgestrick (3), **dadurch gekennzeichnet, dass** das Kompressionsgestrick (3) umfasst:
- einen ungefärbten ersten Grundfaden (4) und einen ungefärbten zweiten Grundfaden (5), wobei der erste Grundfaden (4) zumindest an seiner Oberfläche aus einer Polyamid-Kunstfaser besteht, die einen ersten Farbstoff annimmt, einen zweiten Farbstoff jedoch nicht, und der zweite Grundfaden (5) zumindest an seiner Oberfläche aus einer Polyester-Kunstfaser besteht, die den zweiten Farbstoff annimmt, den ersten Farbstoff jedoch nicht,
- einen als Kompressionsfaden (6) ausgebildeten ungefärbten Schussfaden.

2. Kompressionsgestrickteil (1) nach Anspruch 1, **dadurch gekennzeichnet, dass** der Kompressionsfaden (6) einen mit derselben Kunstfaser wie einer der Grundfäden (4, 5), umwundenen Fadenkern umfasst.

3. Kompressionsgestrickteil (1) nach Anspruch 1 oder 2, **dadurch gekennzeichnet, dass** der Kompressionsfaden (6) einen mit der Polyamid-Grundfaser des ersten Grundfadens (4) umwundenen Fadenkern, insbesondere aus Elastan, umfasst.

4. Kompressionsgestrickteil (1) nach Anspruch 2 oder 3, **dadurch gekennzeichnet, dass** der Kompressionsfaden (6) eine unterschiedliche Farbstoffaffinität als der dieselbe Kunstfaser aufweisende Grundfaden (4, 5) bezüglich des entsprechenden Farbstoffs aufweist, so dass bei Färben ein Ton-in-Ton-Effekt hinsichtlich des entsprechenden Farbstoffs entsteht.

5. Kompressionsgestrickteil (1) nach Anspruch 4, **dadurch gekennzeichnet, dass** die unterschiedliche Farbstoffaffinität durch eine unterschiedliche Oberflächenstruktur des Kompressionsfadens (6) im Vergleich zu dem dieselbe Kunstfaser aufweisenden Grundfaden (4, 5) realisiert ist.

6. Kompressionsgestrickteil (1) nach einem der vorangehenden Ansprüche, **dadurch gekennzeichnet, dass** wenigstens einer der Grundfäden (4, 5) einen mit der entsprechenden Kunstfaser umwundenen Fadenkern, insbesondere aus Elastan, aufweist und/oder dass die Polyester-Kunstfaser aus kationisch färbbarem Polyester besteht.

7. Kompressionsgestrickteil (1) nach einem der vorangehenden Ansprüche, **dadurch gekennzeichnet, dass** einer der Grundfäden (4, 5) ein mustergebender Musterfaden ist, der auf einer Sichtseite des Kompressionsgestricks (3) vor dem anderen Grundfaden (5, 4) liegt.

8. Kompressionsgestrickteil (1) nach Anspruch 7, **dadurch gekennzeichnet, dass** der den Musterfaden bildende Grundfaden (4, 5) in einer Rechts/Links-Grundbindung und der andere Grundfaden (5, 4) in einer Rechts/Rechts-Grundbindung gestrickt sind.

9. Kompressionskleidungsstück, umfassend ein Kompressionsgestrickteil (1) nach einem der vorangehenden Ansprüche.

10. Verfahren zum Färben eines Kompressionsgestrickteils (1) nach einem der Ansprüche 1 bis 8 in einem einbadigen Färbevorgang, umfassend folgende Schritte:
- Vorbehandlung des Kompressionsgestrickteils (1),
- Zugabe des ersten Farbstoffs für die Polyamid-Kunstfasern in eine verwendete Flotte und Färben des Kompressionsgestrickteils (1) bei wenigstens einer ersten Temperatur,
- Nach Ablauf einer ersten Färbezeitspanne Zugabe des zweiten Farbstoffs einer von der Farbe des ersten Farbstoffs unterschiedlichen Farbe zu der Flotte und Aufheizen der Flotte,
- Färben bei wenigstens einer zweiten Temperatur, die höher ist als die erste
Temperatur, aber gleich oder kleiner 97 °C ist, in einer zweiten Färbezeitspanne,
- Nachbehandlung des Kompressionsgestrickteils (1),
wobei zur Trennung des ersten und des zweiten Farbstoffs ein Farbstofftrennmittel verwendet wird.

11. Verfahren nach Anspruch 10, **dadurch gekennzeichnet, dass** als erster Farbstoff ein anionischer Farbstoff zur Färbung der Polyamid-Kunstfasern und als zweiter Farbstoff ein kationischer Farbstoff zur Färbung der kationisch färbbar ausgebildeten Polyester-Kunstfasern verwendet wird und/oder als Farbstofftrennmittel ein Egalisierungsmittel verwendet wird.

12. Verfahren zur Herstellung eines gefärbten Kompressionsgestrickteils (1), umfassend die Schritte:
- Bereitstellen und Lagern eines ungefärbten Kompressionsgestrickteils (1) nach einem der Ansprüche 1 bis 8,
- Bei Erhalt einer zwei zu verwendende Farben beschreibenden Farbanforderung Färben des Kompressionsgestrickteils (1) entsprechend der Farbanforderung in einem Verfahren nach einem der Ansprüche 10 oder 11.

## Claims

1. Compression knit part (1) consisting of a compression knit (3), **characterized in that** the compression knit (3) comprises:
- an undyed first ground thread (4) and an undyed second ground thread (5), wherein the first ground thread (4) consists at least at its surface of a manufactured polyamide fiber that accepts a first dye but not a second dye, and the second ground thread (5) consists at least at its surface of a manufactured polyester fiber which accepts the second dye but not the first,
- an undyed weft thread configured as compression thread (6).

2. Compression knit part (1) according to Claim 1, **characterized in that** the compression thread (6) comprises a thread core, overwrapped with the same manufactured fiber as one of the ground threads (4, 5).

3. Compression knit part (1) according to Claim 1 or 2, **characterized in that** the compression thread (6) comprises a thread core, especially of elastane, overwrapped with the polyamide ground fiber of the first ground thread (4).

4. Compression knit part (1) according to Claim 2 or 3, **characterized in that** the compression thread (6) has a different dye affinity from the ground thread (4, 5) including the same manufactured fiber, in respect of the corresponding dye, to create on dyeing a tone-on-tone effect with regard to the corresponding dye.

5. Compression knit part (1) according to Claim 4, **characterized in that** the different dye affinity is realized by a different surface structure of the compression thread (6) as compared with the ground thread (4, 5) including the same manufactured fiber.

6. Compression knit part (1) according to any preceding claim, **characterized in that** at least one of the ground threads (4, 5) includes a thread core, especially of elastane, overwrapped with the corresponding manufactured fiber, and/or **in that** the manufactured polyester fiber consists of cationically dyeable polyester.

7. Compression knit part (1) according to any preceding claim, **characterized in that** one of the ground threads (4, 5) is a pattern-conferring patterning thread which lies in front of the other ground thread (5, 4) on a face side of the compression knit (3).

8. Compression knit part (1) according to Claim 7, **characterized in that** the ground thread (4, 5) forming the patterning thread and the other ground thread (5, 4) are knitted in a plain construction and in a rib construction, respectively.

9. Compression garment comprising a compression knit part (1) according to any preceding claim.

10. Method of dyeing a compression knit part (1) according to any one of Claims 1 to 8 in a one-bath dyeing process comprising the steps of:
- pretreating the compression knit part (1),
- admixing the first dye for the manufactured polyamide fibers into an employed liquor and dyeing the compression knit part (1) at one or more than one first temperature,
- awaiting the end of a first dyeing time span, admixing the second dye in a color other than that of the first dye to the liquor and heating up the liquor,
- dyeing at one or more than one second temperature which is higher than the first temperature but not more than 97°C in a second dyeing time span,
- aftertreating the compression knit part (1), wherein a dye-separating agent is used to separate the first and second dyes.

11. Method according to Claim 10, **characterized in that** the first dye used is an anionic dye for dyeing the manufactured polyamide fibers, the second dye used is a cationic dye for dyeing the cationically dyeable manufactured polyester fibers, and/or the dye-separating agent used is a leveling agent.

12. Method of producing a dyed compression knit part (1), comprising the steps of:
- providing and storing an undyed compression knit part (1) according to any one of Claims 1 to 8,
- receiving a color request describing two colors to be used and dyeing the compression knit part (1) as per the color request in a method according to either of Claims 10 and 11.

## Revendications

1. Élément tricoté de compression (1) à base d'un tricot de compression (3), **caractérisé en ce que** le tricot de compression (3) comprend :
- un premier fil de base non coloré (4) et un deuxième fil de base non coloré (5), le premier fil de base (4) étant constitué au moins sur sa surface par une fibre artificielle de polyamide, qui absorbe un premier colorant mais n'absorbe toutefois pas un deuxième colorant, et le deuxième fil de base (5) étant constitué au moins sur sa surface par une fibre artificielle de polyester, qui absorbe le deuxième colorant, mais n'absorbe toutefois pas le premier colorant,
- un fil de trame non coloré, configuré sous la forme d'un fil de compression (6).

2. Élément tricoté de compression (1) selon la revendication 1, **caractérisé en ce que** le fil de compression (6) comprend un noyau de fil enroulé avec la même fibre artificielle qu'un des fils de base (4, 5).

3. Élément tricoté de compression (1) selon la revendication 1 ou 2, **caractérisé en ce que** le fil de compression (6) comprend un noyau de fil enroulé avec la fibre de base de polyamide du premier fil de base (4), notamment en élasthanne.

4. Élément tricoté de compression (1) selon la revendication 2 ou 3, **caractérisé en ce que** le fil de compression (6) présente une affinité pour les colorants différente de celle du fil de base (4, 5) comprenant la même fibre artificielle, par rapport au colorant en question, de telle sorte qu'un effet ton sur ton soit obtenu au regard du colorant en question lors de la coloration.

5. Élément tricoté de compression (1) selon la revendication 4, **caractérisé en ce que** l'affinité pour les colorants différente est réalisée par une structure de surface différente du fil de compression (6) en comparaison au fil de base (4, 5) comprenant la même fibre artificielle.

6. Élément tricoté de compression (1) selon l'une quelconque des revendications précédentes, **caractérisé en ce qu'**au moins un des fils de base (4, 5) comprend un noyau de fil enroulé avec la fibre artificielle correspondante, notamment en élasthanne, et/ou **en ce que** la fibre artificielle de polyester est constituée par un polyester pouvant être coloré cationiquement.

7. Élément tricoté de compression (1) selon l'une quelconque des revendications précédentes, **caractérisé en ce qu'**un des fils de base (4, 5) est un fil de maille formant un motif, qui est situé sur un côté visible du tricot de compression (3) devant l'autre fil de base (5, 4).

8. Élément tricoté de compression (1) selon la revendication 7, **caractérisé en ce que** le fil de base (4, 5) formant le fil de maille est tricoté selon une armure de base droite/gauche et l'autre de fil de base (5, 4) est tricoté selon une armure de base droite/droite.

9. Vêtement de compression, comprenant un élément tricoté de compression (1) selon l'une quelconque des revendications précédentes.

10. Procédé de coloration d'un élément tricoté de compression (1) selon l'une quelconque des revendications 1 à 8 par un processus de coloration à un bain, comprenant les étapes suivantes :
- le prétraitement de l'élément tricoté de compression (1),
- l'ajout du premier colorant pour les fibres artificielles de polyamide à un bain utilisé et la coloration de l'élément tricoté de compression (1) à au moins une première température,
- après l'écoulement d'une première durée de coloration, l'ajout du deuxième colorant d'une couleur différente de la couleur du premier colorant au bain et le chauffage du bain,
- la coloration à au moins une deuxième température, qui est supérieure à la première température, mais qui est toutefois inférieure ou égale à 97 °C, pendant une deuxième durée de coloration,
- le post-traitement de l'élément tricoté de compression (1),
un agent de séparation de colorants étant utilisé pour la séparation du premier et du deuxième colorant.

11. Procédé selon la revendication 10, **caractérisé en ce qu'**un colorant anionique est utilisé en tant que premier colorant pour la coloration des fibres artificielles de polyamide et un colorant cationique est utilisé en tant que deuxième colorant pour la coloration des fibres artificielles de polyester configurées sous forme colorable cationiquement, et/ou un agent d'égalisation est utilisé en tant qu'agent de séparation de colorants.

12. Procédé de fabrication d'un élément tricoté de compression coloré (1), comprenant les étapes suivantes :
- la préparation et le stockage d'un élément tricoté de compression non coloré (1) selon l'une quelconque des revendications 1 à 8,
- lors de la réception d'une demande de coloration décrivant deux couleurs à utiliser, la coloration de l'élément tricoté de compression (1) conformément à la demande de coloration par un procédé selon l'une quelconque des revendications 10 ou 11.
